# NEW EUROPEAN PATENT SPECIFICATION

(11) **EP 1 437 150 B2**
(45) Date of publication and mention of the opposition decision: **30.06.2021**
(45) Mention of the grant of the patent: 03.07.2013
(21) Application number: 02762777.7
(22) Date of filing: 14.08.2002
(51) Int. Cl.: A61M 5/28, A61M 5/24

(54) **2-CHAMBER TYPE PREFILLED SYRINGE**
VORGEFÜLLTE SPRITZE MIT 2 KAMMERN
SERINGUE PREALABLEMENT REMPLIE COMPORTANT DEUX CHAMBRES

(30) Priority: 21.08.2001 JP 2001249706
(43) Date of publication of application: 14.07.2004
(73) Proprietor: Takeda Pharmaceutical Company Limited, Osaka 541-0045 (JP)
(72) Inventor: TANAKA, Nobuyoshi, Hirakata-shi, Osaka 573-0105 (JP); KATO, Masahiko, Amagasaki-shi, Hyogo 661-0977 (JP)
(74) Representative: Loustalan, Paul William
(86) International application number: PCT/JP2002/008279
(87) International publication number: WO 2003/015854

(56) References cited:
- EP-A1- 0 144 551
- EP-A1- 0 207 544
- EP-A1- 0 599 649
- EP-A1- 0 737 485
- EP-A2- 0 856 324
- EP-A2- 0 856 324
- EP-A2- 0 856 324
- EP-A2- 1 287 841
- WO-A1-95/11051
- WO-A1-95/11051
- DE-A1- 19 912 322
- FR-A1- 2 286 658
- FR-A5- 2 076 853
- GB-A- 705 392
- JP-A- S 625 357
- JP-A- 11 332 984
- JP-U- 3 029 145
- US-A- 2 591 046
- US-A- 5 851 200
- US-A- 5 865 804

## Description

### BACKGROUND OF THE INVENTION

### Technical Field

The present invention relates to a dual-chamber type prefilled syringe comprising a cylindrical member which has a base end portion formed with an insertion inlet for a plunger rod and a frontend portion provided with an injection needle attaching portion, an end plug member being inserted and fitted into a side of the base end portion, a middle plugmember being arranged between the front end portion and the end plug member, the cylindrical member having an interior area hermetically partitioned into a front chamber on a side of the front end portion and a rear chamber on the side of the base end portion, the cylindrical member having an inner surface between the front end portion and the middle plug member, projected outwards to form a bypass in the shape of a groove, the bypass having a length in a direction of an axis of the cylindrical member, which is made longer than the middle plug member.

### Prior Art

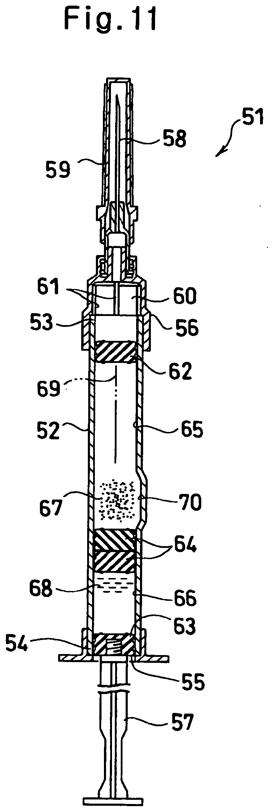

There is a conventional example of the dual-chamber type prefilled syringe disclosed in Japanese Patent Public Disclosure No. 62-5357, which is provided with two chambers formed within a cylindrical member by a plurality of plug members. More specifically, as shown in Fig. 11, this conventional technique inserts and fits a front plug member 62 into a side of a front end portion 53 with an injection needle attaching portion 56 of a cylindrical member 52 and an end plug member 63 into a side of a base end portion 54 formed with an insertion inlet 55 for a plunger rod 57. A middle plug member 64 is arranged between both of the plug members 62 and 63 to hermetically partition an interior area of the cylindrical member 52 into a front chamber 65 on the side of the front end portion 53 and a rear chamber 66 on the side of the base end portion 54. An injection needle 58 is attached to the injection needle attaching portion 56 and is covered with a protector cap 59.

The cylindrical member 52 has an inner surface between the front plug member 62 and the middle plug member 64, formed with a bypass 70 projecting outwards and shaped like a groove. This bypass 70 has a length in a direction of an axis 69 of the cylindrical member, which is longer than the middle plug member 64. The front chamber 65 contains, e.g., powdered medicine 67 and the rear chamber 66 accommodates dissolving solution or the like liquid agent 68, respectively and hermetically.

As for the conventional prefilled syringe 51, when advancing the end plug member 63 by pushing forward the plunger rod 57, the middle plug member 64 advances with an inner pressure of the liquid agent 68 hermetically enclosed in the rear chamber 66. The front plug member 62 also advances during an initial term of the forward pushing of the plunger rod 57. The injection needle attaching portion 56 has an interior area formed with a plug member accommodating portion 60, which has an inner peripheral wall concaved to provide communication grooves 61. Thus if the front plug member 62 advances to enter into the plug member accommodating portion 60, the front chamber 65 communicates with the injection needle 58 via the communication grooves 61 and a clearance between the front plug member 62 and an inner surface of the plug member accommodating portion 60. In this state, if the plunger rod 57 is further pushed forward, air within the front chamber 65 is discharged out of the injection needle 58 and the middle plug member 64 advances to reach a position where the bypass 70 is formed. This allows the rear chamber 66 and the front chamber 65 to communicate with each other through the bypass 70. Therefore, when the plunger rod 57 is pushed forward, the liquid agent 68 within the rear chamber 66 flows into the front chamber 65 through the bypass 70. Then the powdered medicine 67 is suspended or dissolved in the flowed-in liquid agent 68.

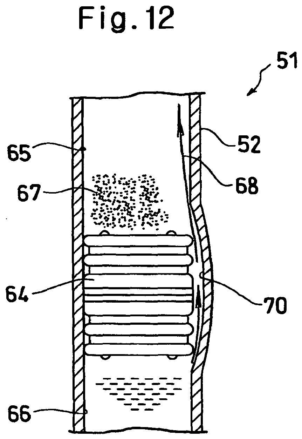

According to the foregoing conventional technique, during an initial term of the communication between the rear chamber 66 and the front chamber 65, the liquid agent 68 which passes through the bypass 70 owns so large a kinetic energy that for example, as shown in Fig. 12, this liquid agent 68 passes through and splashes out of the bypass 70 as if it were a water pistol. In the case where the liquid agent 68 splashes too vigorously, there is a likelihood that it reaches the front end portion 53 and collides against a rear surface of the front plug member 62 within the plug member accommodating chamber 60 to flow into the communication grooves 61 and the clearance between the front plug member 62 and the inner surface of the plug member accommodating portion 60. Especially, if the plunger rod 57 is pushed forward acceleratedly in order to carry out this communicating operation quickly, the liquid agent 68 splashes out more vigorously to flow into the grooves 61 and the clearance more easily.

And once the liquid agent 68 has entered the communication grooves 61 or the like, it cannot readily return to the interior area of the front chamber 65, so that if the plunger rod 57 is pushed forward thereafter, it is pushed out with the air within the front chamber 65 to leak out of the injection needle 58. As a result, there was caused not only a likelihood of dirtying or damaging the surroundings of the dual-chamber type prefilled syringe 51 by the liquid agent 68 but also a fear of shortage in a liquid amount required for dissolving the powdered medicine 67 within the front chamber 65 to result in inadequate dissolution.

The water pistol phenomenon has also occurred in the dual-chamber type prefilled syringe which does not use the front plug member. Accordingly, there was a likelihood that the liquid agent which splashed out of the bypass flowed into a communication passage between the bypass and the injection needle to have leaked out of the injection needle.

A further example of a dual-chamber type prefilled syringe is to be found in International application WO 95/11051.

The present invention seeks to reduce the so-called water pistol phenomenon in which the dissolving solution or the like liquid agent splashes out of the bypass when communicating the front chamber with the rear chamber to prevent the liquid agent from leaking out of the front end of the injection needle.

The dual-chamber-type prefilled syringe in accordance with the invention is characterised in that the bypass has an inner surface an end surface of which is situated on aside of an inlet formed on the side of the base end portion and uprises outwards by an angle which is made larger than 45 degrees with respect to the axis of the cylindrical member.

### Disclosure of the Invention

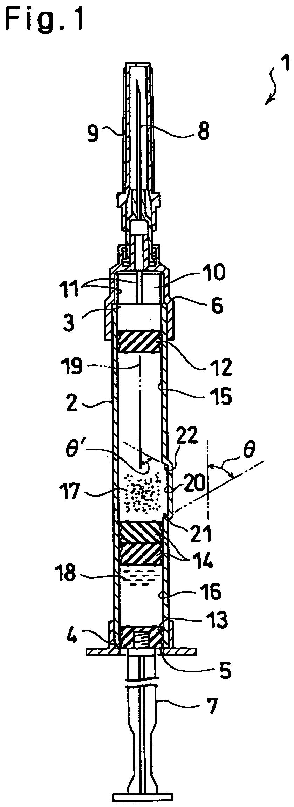

Figs. 1 to 10 show embodiments of the present invention.

A first embodiment fits and inserts an end plug member 13 into a side of a base end portion 4 formed with an insertion inlet 5 for a plunger rod 7, of a cylindrical member 2. A middle plug member 14 is arranged between the end plug member 13 and a front end portion 3 provided with an injection needle attaching portion 6. The cylindrical member 2 has an interior area hermetically partitioned into a front chamber 15 on a side of the front end portion 3 and a rear chamber 16 on the side of the base end portion 4. The cylindrical member 2 has an inner surface between the front end portion 3 and the middle plug member 14, provided with a bypass 20 which projects outwards and is shaped like a groove. This bypass 20 has a length in a direction of an axis 19 of the cylindrical member 2, which is longer than the middle plug member 14. The bypass 20 has an inner surface an end surface of which is positioned on a side of an inlet 21 formed on the side of the base end portion 4. The end surface uprises outwards by an angle (θ) which is larger than 45 degrees with respect to the axis 19.

Owing to the foregoing construction, the present invention offers the following advantages.

Since the bypass has the end surface on the inlet side made to uprise at an angle larger than 45 degrees, the liquid agent which flows from the rear chamber into this bypass is directed largely outwards and collides against a groove bottom surface of the bypass to have part of its kinetic energy absorbed. As a result, it is possible to reduce the so-called water pistol phenomenon in which the dissolving solution or the like liquid agent splashes out of the bypass and to prevent the liquid agent which has splashed out of the bypass from reaching the front end portion of the cylindrical member. And eventually it is possible to inhibit the liquid agent from leaking out of the front end of the injection needle when communicating the front chamber with the rear chamber.

Further, thanks to the fact that the fact that the bypass has the end surface on the inlet side made to uprise at a large angle, a slight advancement of the middle plug member rapidly increases the clearance formed between the middle plug member and an inner surface of the bypass to result in abruptly decreasing a flow speed of the liquid agent which flows from the rear chamber into the bypass, which can in turn more effectively suppress the water pistol phenomenon.

In addition, a second embodiment inserts and fits the end plug member 13 into the side of the base end portion 4 formed with the insertion inlet 5 of the plunger rod 7. The middle plug member 14 is arranged between the end plug member 13 and the front end portion 3 provided with the injection needle attaching portion 6. The cylindrical member 2 has the interior area hermetically partitioned into the front chamber 15 on the side of the front end portion 3 and the rear chamber 16 on the side of the base end portion 4. The cylindrical member 2 has the inner surface between the front end portion 3 and the middle plug member 14, formed with the bypass 20 which projects outwards and is shaped like a groove. The bypass 20 has a length in the direction of the axis 19 of the cylindrical member 2, which is longer than the middle plug member 14. The bypass 20 has a longitudinal direction inclined with respect to the axis 19 of the cylindrical member 2. Further, the bypass has the longitudinal direction inclined with respect to the axis of the cylindrical member by an angle which is preferably set to at least 10 degrees, more preferably at least 20 degrees, and much more preferably at least 25 degrees.

The above construction offers the following advantages.

Since the bypass has the longitudinal direction inclined with respect to the axis of the cylindrical member, the liquid agent which flows from the rear chamber into the bypass collides against a lateral surface on the side of the front end portion of the bypass inner surface and further the liquid agent which flows from the bypass into the front chamber circulates along the inner surface of the cylindrical member to have part of its kinetic energy absorbed. As a result, it is possible to reduce the so-called water pistol phenomenon in which the dissolving solution or the like liquid agent splashes out of the bypass and to inhibit the liquid agent which has splashed out of the bypass from reaching the front end portion of the cylindrical member, which can in turn prohibit the liquid agent from leaking out of the front end of the injection needle when communicating the front chamber with the rear chamber.

Moreover, the liquid agent which has flowed out of the bypass obliquely circulates spirally, so that a distance along a direction in which the liquid agent circulates until it reaches the front end portion of the cylindrical member becomes greater than a distance of the axial direction of the cylindrical member. This results in preventing the liquid agent from arriving at the front end portion, thereby more effectively inhibiting the leakage of the liquid agent from the front end of the injection needle.

A third embodiment fits and inserts the end plug member 13 into the side of the base end portion 4 formed with the insertion inlet 5 for the plunger rod 7. The middle plug member 14 is arranged between the end plug member 13 and the front end portion 3 provided with the injection needle attaching portion 6. The cylindrical member 2 has the interior area hermetically partitioned into the front chamber 15 on the side of the front end portion 3 and the rear chamber 16 on the side of the base end portion 4. The cylindrical member 2 has the inner surface between the front end portion 3 and the middle plug member 14, formed with the bypass 20 projecting outwards and shaped like a groove. This bypass 20 has a length in the direction of the axis 19 of the cylindrical member 2, which is longer than the middle plug member 14. The bypass 20 has a mid portion provided with a bent portion 23. The bent portion 23 may be provided at a portion of the bypass, for example, in the shape of an angled 'C' or at a plurality of portions thereof.

The foregoing construction offers the following advantages.

Since the bypass has the mid portion provided with the bent portion, the liquid agent which flows from the rear chamber into the bypass collides against the inner surface of the bypass at the bent portion when it passes through the bypass to result in having its part of kinetic energy absorbed. As a result, it is possible to reduce the so-called water pistol phenomenon in which the dissolving solution or the like liquid agent splashes out of the bypass and to prevent the liquid agent which has splashed out of the bypass from reaching the front end portion of the cylindrical member, which can in turn inhibit the leakage of the liquid agent from the front end of the injection needle when communicating the front chamber with the rear chamber.

A fourth embodiment fits and inserts the end plug member 13 into the side of the base end portion 4 formed with the insertion inlet 5 for the plunger rod 7. The middle plug member 14 is arranged between the end plug member 13 and the front end portion 3 provided with the injection needle attaching portion 6. The cylindrical member 2 has the interior area hermetically partitioned into the front chamber 15 on the side of the front end portion 3 and the rear chamber 16 on the side of the base end portion 4. The cylindrical member 2 has the inner surface between the front end portion 3 and the middle plug member 14, formed with a plurality of bypasses 20 each projecting outwards and shaped like a groove. These bypasses 20 include a first bypass 20a on the side of the base end portion 4 and a second bypass 20b on the side of the front end portion 3. The first bypass 20a has a length in a direction of the axis 19 of the cylindrical member 2, which is shorter than the middle plug member 14. A length in the direction of the axis 19 of the cylindrical member 2 from an inlet 21a of the first bypass 20a to an outlet 22b of the second bypass 20b is longer than the middle plug member 14. The middle plug member 14 has an outer peripheral surface concaved to form a groove 24 which communicates the first bypass 20a and the second bypass 20b with each other when the middle plug member 14 has moved to a position where the bypasses 20 are formed.

The foregoing construction offers the following advantages.

When the middle plug member has reached the position where the bypasses are formed, the rear chamber communicates with the front chamber via the first bypass, the concaved groove and the second bypass in the mentioned order. And the liquid agent which has flowed from the rear chamber into the first bypass collides against the end surface on the outlet side of the first bypass to flow into the concaved groove and collide against an inner surface of the same. Further, it flows into the second bypass to collide against an inner surface of the second bypass and then flow into the front chamber. Thus, when the liquid agent collides against the end surface on the outlet side of the first bypass, the inner surface of the concaved groove and the inner surface of the second bypass, it has part of its kinetic energy absorbed. As a result, it is possible to reduce the so-called water pistol phenomenon in which the dissolving solution or the like liquid agent splashes out of the bypass and to prevent the liquid agent splashed out of the bypass from reaching the front end portion of the cylindrical member, which in turn can inhibit the leakage of the liquid agent from the front end of the injection needle when communicating the front chamber with the rear chamber.

In any one of the second to the fourth embodiments, the bypass 20 has the inner surface the end surface of which is situated on the side of the inlet 21 formed on the side of the base end portion 4 and uprises outwards by an angle (θ) which can be formed larger than 45 degrees with respect to the axis 19 of the cylindrical member 2 as well as in the first invention.

Further, in each of the foregoing embodiments, the bypass 20 has the inner surface an end surface of which is situated on a side of an outlet 22 formed on the side of the front end portion 3 and uprises outwards by an angle (θ') which can be formed larger than 45 degrees with respect to the axis 19 of the cylindrical member 2. In this case, since the end surface on the outlet side of the bypass uprises at an angle larger than 45 degrees, the liquid agent which passes through the bypass straightly collides against this outlet side end surface to have part of its kinetic energy absorbed and then flows into the front chamber. As a result, it is possible to more effectively reduce the phenomenon in which the dissolving solution or the like liquid agent splashes out of the bypass and to prevent the liquid agent splashed out of the bypass from reaching the front end portion of the cylindrical member 2, which in turn more effectively can inhibit the leakage of the liquid agent from the front end of the injection needle when communicating the front chamber with the rear chamber.

In each of the above-mentioned embodiments, the inlet side end surface or the outlet side end surface of the bypass uprises at an angle preferably set to at least 50 degrees, more preferably to at least 60 degrees. The uprising angle means an average angle at the mid portion of the end surface. Accordingly, needless to say, the end surface may be connected to the inner surface of the cylindrical member or to the groove bottom portion of the bypass, by a portion which consists of a smooth curve.

### Brief Description of the Drawings

Figs. 1 to 3 show a first embodiment of the present invention. Fig. 1 is a sectional view of a dual-chamber type prefilled syringe. Fig. 2 shows, in an enlarged section, the neighborhood of a bypass when conducting a communication operation. Fig. 3 is a front view of the bypass;

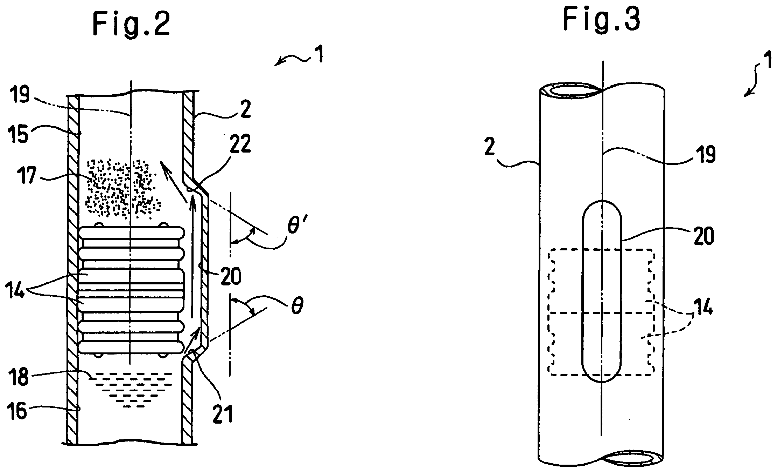

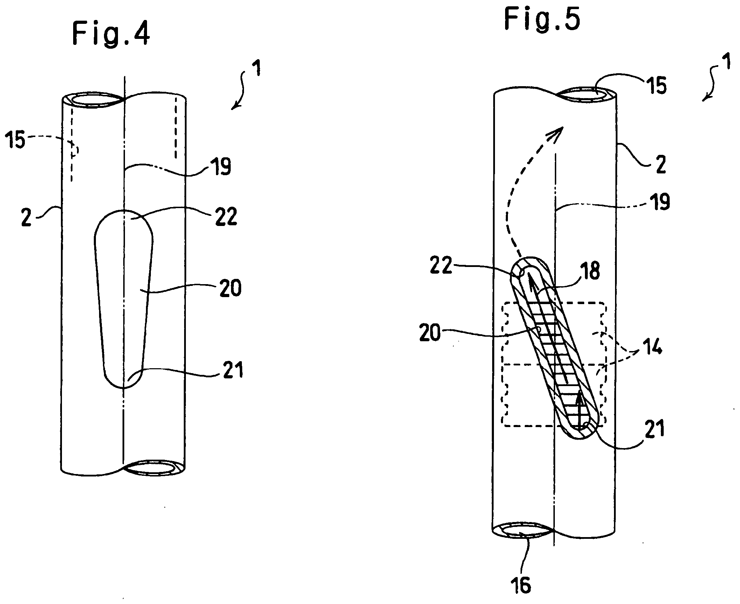

Fig. 4 shows a modification of the first embodiment and is similar to Fig. 3;
Fig. 5 shows a second embodiment of the present invention and is a partly broken front view illustrating the neighborhood of the bypass of the dual-chamber type prefilled syringe;

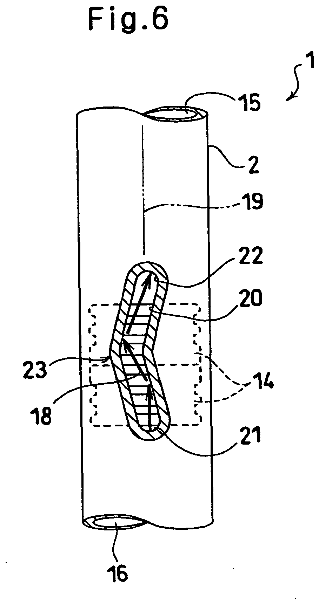

Fig. 6 shows a third embodiment of the present invention and is a partly broken front view illustrating the neighborhood of the bypass of the dual-chamber type prefilled syringe;

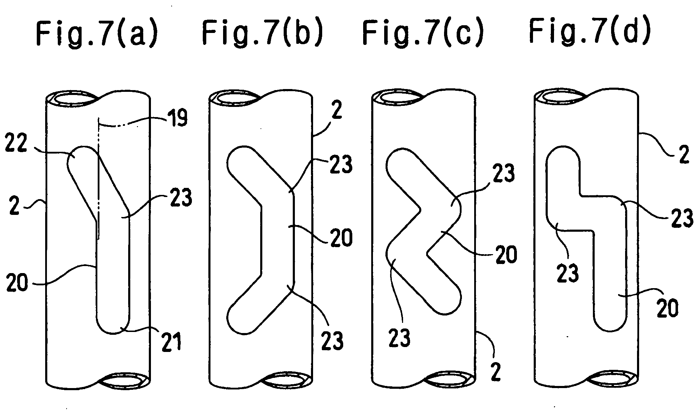

Fig. 7 shows respective modifications of the third embodiment of the present invention. Figs. 7(a) to 7(d) are front views illustrating the bypass portions of a first to a fourth modifications of the third embodiment, respectively;

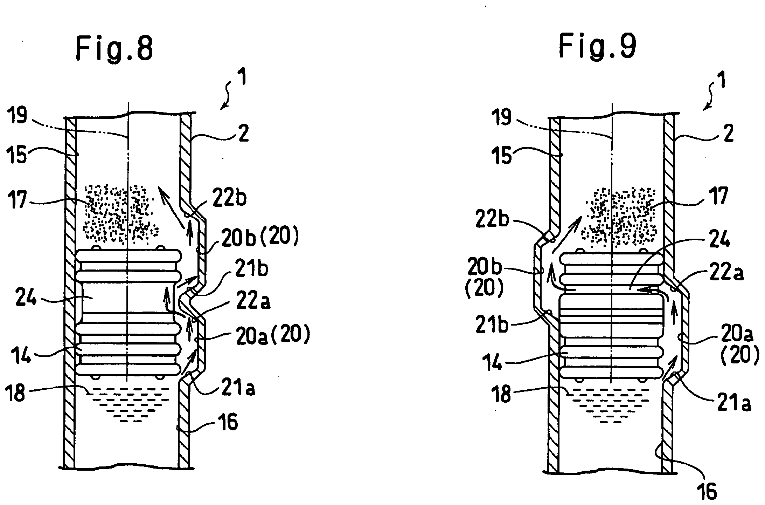

Fig. 8 shows a fourth embodiment of the present invention and illustrates, in an enlarged section, the neighborhood of the bypass of the dual-chamber type prefilled syringe when conducting the communicating operation;
Fig. 9 shows a first modification of the fourth embodiment and is similar to Fig. 8;

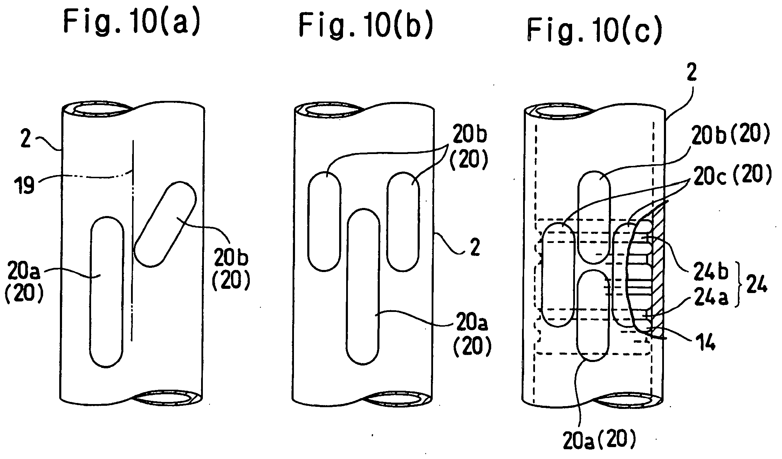

Fig. 10 show other modifications of the fourth embodiment. Figs. 10(a) and 10(b) are front views of the bypass portions of a second and a third modifications of the fourth embodiment, respectively. Fig. 10(c) is a partly broken front view showing the bypass portion of a fourth modification of the fourth embodiment when carrying out the communicating operation; and
Figs. 11 and 12 show prior art. Fig. 11 shows a dual-chamber type prefilled syringe and is similar to Fig. 1. Fig. 12 is a view similar to Fig. 2.

### Most Preferred Embodiments of the Invention

Hereafter, an explanation is given for the embodiments of the present invention based on the attached drawings.

Figs. 1 to 3 show a first embodiment. Fig. 1 is a sectional view of a dual-chamber type prefilled syringe. Fig. 2 illustrates, in an enlarged section, the neighborhood of a bypass when conducing a communicating operation. Fig. 3 is a front view of the bypass portion.

As shown in Fig. 1, this dual-chamber type prefilled syringe 1 comprises a cylindrical member 2 made of glass or plastics, which is provided at its front end portion 3 with an injection needle attaching portion 6 and at its base end portion 4 with an insertion inlet 5 for a plunger rod 7.

The injection needle attaching portion 6 has a front end to which an injection needle 8 is attached. A protector cap 9 is covered around the injection needle 8. Further, the injection needle attaching portion 6 has an interior area formed with a plug member accommodating portion 10 which has an inner peripheral wall concaved to provide communication grooves 11.

The cylindrical member 2 has the front end portion 3 into a side of which a front plug member 12 is inserted and fitted and has a base end portion into a side of which an end plug member 13 is inserted and fitted. A middle plug member 14 is arranged between the both plug members 12 and 13. The cylindrical member 2 has an interior area hermetically partitioned into a front chamber 15 on the side of the front end portion 3 and a rear chamber 16 on the side of the base end portion 4. And the front chamber 15 accommodates, for example, powdered medicine 17 and the rear chamber 16 contains dissolving solution or the like liquid agent 18, respectively and hermetically.

It is to be noted that although in this embodiment, the front chamber accommodates the powdered medicine and the rear chamber contains dissolving solution or the like liquid agent, this prefilled syringe may contain liquid medicine in the front chamber and a second liquid medicine in the rear chamber.

In addition, in this embodiment, the middle plug member 14 is composed of a plug member on the side of the powdered medicine and another plug member on the side of the liquid agent. However, needless to say, according to the present invention, the middle plug member may be composed of a single plug member.

The cylindrical member 2 has an inner surface between the front plug member 12 and the middle plug member 14, projected outwards to form a bypass 20 in the shape of a groove. This bypass 20 has a length in a direction of an axis 19 of the cylindrical member 2, which is longer than the middle plug member 14.

As shown in Fig. 2, the bypass 20 has an inner surface an end surface of which is situated on a side of an inlet 21 formed on the side of the base end portion 4 and uprises outwards by an angle (θ) which is formed larger than 45 degrees, for example, about 60 degrees with respect to the axis 19 of the cylindrical member 2. Besides, an end surface on a side of an outlet 22 formed on the side of the front end portion 3 of the bypass 20 uprises outwards by an angle (θ') which is also set to about 60 degrees with respect to the axis 19 of the cylindrical member 2. As for the respective uprising angles (θ and θ'), the larger the better. But when taking into consideration the readiness of forming the bypass 20 and the smoothness of an outer surface of the cylindrical member 2, they are generally formed within a range of 50 degrees to 70 degrees.

An outer appearance of the bypass 20, as shown in Fig. 3, is formed so as to have substantially a constant width along the axis 19 of the cylindrical member 2. However, the bypass 20 of the present invention is not limited to the shape of the present embodiment. For instance, like a modification shown in Fig. 4, a groove width may be formed larger on the side of the outlet 22 than on the side of the inlet 21. For example, the largest groove width on the side of the outlet 22 may be formed 1.2 times to 5 times a width on the side of the inlet 21. In this case, since a flow passage of the liquid agent within the bypass 20 is enlarged, the liquid agent flows from the bypass 20 into the front chamber 15 at a reduced speed, which is more preferable.

Next, an explanation is given for a communicating operation in which the front chamber is communicated with the rear chamber through the bypass and the liquid agent is flowed into the front chamber to dissolve or suspend the powdered medicine.

Initially, the plunger rod 7 has its front end engaged in screw-thread relationship with the end plug member 13 and is pushed forward, thereby advancing the end plug member 13 to advance the middle plug member 14 with an inner pressure of the liquid agent 18 hermetically contained in the rear chamber 16. Further, with a pressure in the front chamber 15 increased, the front plug member 12 also advances. When this front plug member 12 advances and enters into the plug member accommodating portion 10, the front chamber 15 communicates with the injection needle 8 via the communication grooves 11 and a clearance between the front plug member 12 and an inner surface of the plug member accommodating portion 10.

On further pushing forward plunger rod 7 in this state, air within the front chamber 15 is discharged out of the injection needle 8 and the middle plug member 14 advances to reach a position where the bypass 20 is formed, as shown in Fig. 2.

When a rear end of the middle plug member 14 advances over the end surface on the side of the inlet 21 of the bypass 20, the rear chamber 16 communicates with the front chamber 15 through the bypass 20, so that the liquid agent 18 within the rear chamber 16 tries to flow into the front chamber vigorously via the bypass 20 if the plunger rod 7 is pushed forward.

At this time, the liquid agent 18 which flows from the rear chamber 16 into the bypass 20 is oriented outwards largely because the end surface on the side of the inlet 21 of the bypass 20 uprises at an angle (θ) formed to about 60 degrees, and it collides against the groove bottom surface of the bypass 20 to have part of its kinetic energy absorbed. Further, this liquid agent 18 passes through the bypass 20 straightly. However, the end surface on the side of the outlet 22 also uprises at an angle (θ') formed to about 60 degrees, so that it collides against this end surface as well to have also part of its kinetic energy absorbed at this time. Meanwhile, since the end surface on the side of the inlet 21 uprises at a large angle (θ) to result in quickly enlarging the clearance between the middle plug member 14 and the inner surface of the bypass 20 through even a slight advancement of the middle plug member 14, the liquid agent 18 flows from the rear chamber 16 into the bypass 20 at a abruptly reduced speed. As a result, the liquid agent 18 moderately flows and enters from the bypass 20 into the front chamber 15.

When pushing the plunger rod 7 forward to further advance the end plug member 13, almost whole amount of the liquid agent 18 within the rear chamber 16 flows into the front chamber 15 via the bypass 20 to bring the end plug member 13 into contact with the middle plug member 14.

On further pushing the plunger rod 7 forward to advance the end plug member 13, the middle plug member 14 has a front end advanced ahead of the outlet 22 of the bypass 20 to clog the bypass 20. This terminates the communicating operation. In this state, if the dual-chamber type prefilled syringe 1 is shook or the like, the powdered medicine 17 is suspended or dissolved in the liquid agent 18 to complete the preparation for administering the medicine.

Fig. 5 shows a second embodiment of the present invention and is a partly broken front view of the neighborhood of the bypass of the dual-chamber type prefilled syringe.

In this second embodiment, the cylindrical member 2 is formed with the bypass 20 in the shape of a groove, which projects outwards and has its longitudinal direction inclined by an angle of about 20 degrees with respect to the axis 19 of the cylindrical member 2. The bypass 20 has a length in the direction of the axis 19 of the cylindrical member 2, which is made longer than the middle plug member 14.

The other construction is the same as that of the first embodiment and therefore we refrain from explaining it.

In this second embodiment, like in the first embodiment, the middle plug member 14 reaches the position where the bypass 20 is formed, on conducting the communicating operation. When the middle plug member 14 has its rear end advanced ahead of the inlet 21 of the bypass 20, the rear chamber 16 communicates with the front chamber 15 through the bypass 20.

Thus the liquid agent 18 within the rear chamber 16 flows into the front chamber 15 via the bypass 20. However, at this time, since the bypass 20 has its longitudinal direction inclined with respect to the axis 19 of the cylindrical member 2, the liquid agent 18 which flows into the bypass 20 collides against a lateral surface which is situated on the side of the front end portion, of the inner surface of the bypass 20 to have part of its kinetic energy absorbed. In addition, the liquid agent 18 which flows from the bypass 20 into the front chamber 15 is circulated along the inner surface of the cylindrical member 2, thereby having part of its kinetic energy absorbed as well.

Moreover, the liquid agent 18 which flows out of the bypass 20 circulates spirally, so that it reaches the front plug member at the front end portion 3 by a distance which is longer than that by which it goes straight along the direction of the axis 19 of the cylindrical member 2. As a result, it is possible to prevent the liquid agent 18 which flows out of the bypass 20 from arriving at the front end portion

Fig. 6 shows a third embodiment of the present invention and is a partly broken front view of the neighborhood of the bypass of the dual-chamber type prefilled syringe.

In this third embodiment, the cylindrical member 2 is formed with the bypass 20 in the shape of the groove, which is provided at its mid portion with a bent portion 23 formed in the shape of an angled 'C'. And the bypass 20 has a length in the direction of the axis 19 of the cylindrical member, which is longer than the middle plug member 14.

The other construction is the same as that of the first embodiment and therefore we refrain from explaining it.

Also in this third embodiment, like in the first embodiment, the middle plug member 14 reaches the position where the bypass 20 is formed, on conducting the communicating operation. When the middle plug member 14 has its rear end advanced ahead of the inlet 21 of the bypass 20, the rear chamber 16 communicates with the front chamber 15 through this bypass 20.

Thus the liquid agent 18 within the rear chamber 16 flows into the front chamber 15 via the bypass 20. However, at this time, the liquid agent 18 collides against the inner surface of the bypass 20 at the bent portion 23 to have part of its kinetic energy absorbed.

Further, in this third embodiment, as shown in Fig. 6, the bypass 20 is inclined with respect to the axis 19 of the cylindrical member on the side of the outlet 22 and the liquid agent 18 which flows from the bypass 20 into the front chamber 15 is circulated along the inner surface of the cylindrical member 2 as well as in the second embodiment, thereby having part of its kinetic energy absorbed. Besides, since the liquid agent 18 which has flowed into the front chamber 15 circulates along the inner surface of the cylindrical member 2, it reaches the front plug member at the front end portion by a distance longer than that by which it goes straight along the direction of the axis 19 of the cylindrical member 2 as in the second embodiment. From this point of view, it is possible to prevent the liquid agent 18 which flows out of the bypass 20 from arriving at the front end portion of the cylindrical member.

In the foregoing third embodiment, the bypass is formed in the shape of the angled 'C'. However, the above-mentioned bent portion may be provided at optional one or more than one positions at the mid portion of the bypass like the respective modifications as shown in Fig. 7.

More specifically, the first modification shown in Fig. 7(a) is the same as the third embodiment in that the bent portion 23 is provided at one position of the mid portion of the bypass 20. But the bypass 20 has a portion extending from this bent portion 23 toward the side of the inlet 21, formed along the axis 19 of the cylindrical member 2 and has another portion which extends from the bent portion 23 toward the side of the outlet 22, inclined with respect to the axis 19 of the cylindrical member 2. Moreover, each of the second to fourth modifications shown in Figs. 7(b) to 7(d) is formed with bent portions (23, 23) at two positions of the bypass 20.

Fig. 8 shows a fourth embodiment and is a sectional view of the neighborhood of the bypass of the dual-chamber type prefilled syringe.

In this fourth embodiment, the cylindrical member 2 is formed with two bypasses 20 which consist of a first bypass 20a on the side of the base end portion and a second bypass 20b on the side of the font end portion.

The first bypass 20a has a length in the direction of the axis 19 of the cylindrical member 2, which is shorter than the middle plug member 14. A length in the direction of the axis 19 of the cylindrical member 2 which extends from the inlet 21a of the first bypass 20a to the outlet 22b of the second bypass 20b is formed longer than the middle plug member 14.

The middle plug member 14 has a peripheral surface concaved to form a groove 24. When the middle plug member 14 has moved to the position where the bypasses 20 are formed, the first bypass 20a has the outlet 22a communicated with the inlet 21b of the second bypass 20b through the groove 24.

The other construction is the same as that of the first embodiment and we refrain from explaining it.

In this fourth embodiment, when the middle plug member 14 has reached the position where the bypasses 20 are formed and the middle plug member 14 has a rear end advanced ahead of the inlet 21a of the first bypass 20a on conducing the communication operation, the rear chamber 16 communicates with the front chamber 15 via the first bypass 20a, the concaved groove 24 and the second bypass 20b in the mentioned order.

Thus the liquid agent 18 within the rear chamber 16 flows into the first bypass 20a. However, since the first bypass 20a is shorter than the middle plug member 14, the liquid agent 18 collides against the outlet 22a of the first bypass 20a and flows into the groove 24. And when it flows into the second bypass 20b after it has collided against an inner surface of the groove 24, it also collides against an inner surface of the second bypass 20b and thereafter flows into the front chamber 15. In consequence, the liquid agent 18 has part of its kinetic energy absorbed when it collides against the outlet 22a of the first bypass 20a, the inner surface of the concaved groove 24 and the inner surface of the second bypass 20b to result in flowing moderately and entering the front chamber 15.

In the fourth embodiment, the first bypass 20a is arranged side by side with the second bypass 20b in the direction of the axis 19 of the cylindrical member 2. However, the first bypass 20a may be arranged at a position peripherally different from another position where the second bypass 20b is formed.

For example, in a first modification as shown in Fig. 9, the first bypass 20a is arranged opposite to the second bypass 20b in a peripheral direction of the cylindrical member 2. If they are constructed as such, the inlet 21b of the second bypass 20b can be arranged behind the outlet 22a of the first bypass 20a. Accordingly, the groove 24 formed by concaving the middle plug member 14 can be made to have a narrow width.

Additionally, the bypass can be modified as shown in Fig. 10.

More specifically, in a second modification as shown in Fig. 10(a), the second bypass 20b has its longitudinal direction inclined with respect to the axis 19 of the cylindrical member 2.

In a third modification as shown in Fig. 10(b), a plurality of second bypasses 20b, 20b are formed on the opposite sides of the first bypass 20a.

In a fourth modification as shown in Fig. 10(c), a plurality of third bypasses 20c, 20c are formed over the opposite sides of the first bypass 20a and the second bypass 20b. In this fourth modification, the groove 24 formed by concaving the outer peripheral surface of the middle plug member 14 consists of a first concaved groove 24a which communicates the first bypass 20a with one third bypass 20c and a second concaved groove 24b which communicates the other third bypass 20c with the second bypass 20b.

In each of the above-mentioned embodiments, the explanation has been given for the dual-chamber type prefilled syringe with the front plug member. However, needless to say, the present invention is applicable to a dual-chamber type prefilled syringe without the front plug member.

## Claims

1. A dual-chamber type prefilled syringe comprising a cylindrical member (2) which has a base end portion (4) formed with an insertion inlet (5) for a plunger rod (7) and a front end portion (3) provided with an injection needle attaching portion (6), an end plug member (13) being inserted and fitted into a side of the base end portion (4), a middle plug member (14) being arranged between the front end portion (3) and the end plug member (13), the cylindrical member (2) having an interior area hermetically partitioned into a front chamber (15) on a side of the front end portion (3) and a rear chamber (16) on the side of the base end portion (4), the cylindrical member (2) having an inner surface and an outer surface between the front end portion (3) and the middle plug member (14), the inner surface and outer surface projected outwards to form a bypass (20) in the shape of a groove, the bypass (20) having a length in a direction of an axis (19) of the cylindrical member (2), which is made longer than the middle plug member (14), the syringe being **characterised in that**
the bypass (20) has an inner surface an end surface of which is situated on a side of an inlet (21) formed on the side of the base end portion (4) and uprises outwards by an angle (θ) which is made larger than 45 degrees with respect to the axis (19) of the cylindrical member (2).

2. A syringe according to claim 1 wherein the bypass (20) has a longitudinal direction inclined with respect to the axis (19) of the cylindrical member (2).

3. A syringe according to claim 1 wherein the bypass (20) has a mid portion provided with a bent portion (23).

4. A syringe according to claim 1 comprising a plurality of bypasses including a first bypass (20a) on the side of the base end portion (4) and a second bypass (20b) on the side of the front end portion (3),
the first bypass (20a) having a length in a direction of an axis (19) of the cylindrical member (2), which is made shorter than the middle plug member (14), a length from an inlet (21a) of the first bypass (20a) to an outlet (22b) of the second bypass (20b) being formed longer than the middle plug member (14),
the middle plug member (14) having an outer peripheral surface concaved to provide a groove (24) which communicates the first bypass (20a) with the second bypass (20b) when the middle plug member (14) has reached a position where the bypasses (20), are formed.

5. A syringe according to any of claims 1 to 4, wherein an end surface of the inner surface of the bypass (20) is situated on a side of an outlet (22) formed on the side of the front end portion (3) and uprises outwards by an angle (θ') which is larger than 45 degrees with respect to the axis (19) of the cylindrical member (2).

## Patentansprüche

1. Vorgefüllte Spritze des Doppelkammertyps, umfassend ein zylindrisches Element (2), das einen Basisendteil (4), der mit einem Einführungseinlass (5) für eine Kolbenstange (7) ausgebildet ist, und einen vorderen Endteil (3), der mit einem Injektionskanülenanbringungsteil (6) versehen ist, hat, ein Endstopfenelement (13), das in eine Seite des Basisendteils (4) eingeführt und eingepasst ist, ein mittleres Stopfenelement (14), das zwischen dem vorderen Endteil (3) und dem Endstopfenelement (13) angeordnet ist, wobei das zylindrische Element (2) einen Innenbereich hat, der hermetisch in eine vordere Kammer (15) auf einer Seite des vorderen Endteils (3) und eine hintere Kammer (16) auf der Seite des Basisendteils (4) unterteilt ist, wobei das zylindrische Element (2) zwischen dem vorderen Endteil (3) und dem mittleren Stopfenelement (14) eine Innenfläche und eine Außenfläche hat, wobei die Innenfläche und die Außenfläche nach außen vorspringen, um eine Umgehung (20) in der Form einer Nut zu bilden, wobei die Umgehung (20) eine Länge in einer Richtung einer Achse (19) des zylindrischen Elements (2) hat, die länger als das mittlere Stopfenelement (14) gemacht ist, wobei die Spritze **dadurch gekennzeichnet** wird, dass:
die Umgehung (20) eine Innenfläche hat, bei der sich eine Endfläche auf einer Seite eines Einlasses (21) befindet, der auf der Seite des Basisendteils (4) ausgebildet ist, und mit einem Winkel (•) aufwärts nach außen verläuft, der größer als 45 Grad in Bezug auf die Achse (19) des zylindrischen Elements (2) gemacht ist.

2. Spritze nach Anspruch 1, wobei die Umgehung (20) eine Längsrichtung hat, die in Bezug auf die Achse (19) des zylindrischen Elements (2) geneigt ist.

3. Spritze nach Anspruch 1, wobei die Umgehung (20) einen Mittelteil hat, der mit einem gebogenen Teil (23) versehen ist.

4. Spritze nach Anspruch 1, die eine Vielzahl von Umgehungen einschließlich einer ersten Umgehung (20a) auf der Seite des Basisendteils (4) und einer zweiten Umgehung (20b) auf der Seite des vorderen Endteils (3) aufweist,
wobei die erste Umgehung (20a) eine Länge in einer Richtung einer Achse (19) des zylindrischen Elements (2) hat, die kürzer als das mittlere Stopfenelement (14) gemacht ist, wobei eine Länge von einem Einlass (21a) der ersten Umgehung (20a) zu einem Auslass (22b) der zweiten Umgehung (20b) länger als das mittlere Stopfenelement (14) ausgebildet ist,
wobei das mittlere Stopfenelement (14) eine äußere Umfangsfläche hat, die zum Bereitstellen einer Nut (24) gewölbt ist, welche die erste Umgehung (20a) mit der zweiten Umgehung (20b) verbindet, wenn das mittlere Stopfenelement (14) eine Position erreicht hat, an der die Umgehungen (20) ausgebildet sind.

5. Spritze nach einem der Ansprüche 1 bis 4, wobei eine Endfläche der Innenfläche der Umgehung (20) sich auf einer Seite eines Auslasses (22) befindet, der auf der Seite des vorderen Endteils (3) ausgebildet ist, und mit einem Winkel (•') aufwärts nach außen verläuft, der größer als 45 Grad in Bezug auf die Achse (19) des zylindrischen Elements (2) ist.

## Revendications

1. Seringue préremplie du type à chambre double comprenant un élément cylindrique (2) qui comporte une partie d'extrémité de base (4) formée avec une entrée d'insertion (5) pour une tige piston (7) et une partie d'extrémité frontale (3) dotée d'une partie de fixation d'aiguille d'injection (6), un élément de bouchon d'extrémité (13) étant inséré et monté dans un côté de la partie d'extrémité de base (4), un élément de bouchon intermédiaire (14) étant agencé entre la partie d'extrémité frontale (3) et l'élément de bouchon d'extrémité (13), l'élément cylindrique (2) ayant une zone intérieure hermétiquement compartimentée en une chambre frontale (15) sur un côté de la partie d'extrémité frontale (3) et une chambre arrière (16) sur le coté de la partie d'extrémité de base (4), l'élément cylindrique (2) ayant une surface interne et une surface externe entre la partie d'extrémité frontale (3) et l'élément de bouchon intermédiaire (14), la surface interne et la surface externe étant projetées vers l'extérieur pour former une dérivation (20) sous forme de rainure, la dérivation (20) ayant une longueur dans un sens d'un axe (19) de l'élément cylindrique (2), laquelle est rendue plus longue que l'élément de bouchon intermédiaire (14), la seringue étant **caractérisée en ce que**
la dérivation (20) a une surface interne dont une surface d'extrémité est située sur un côté d'une entrée (21) formée sur le côté de la partie d'extrémité de base (4) et s'élève vers l'extérieur par un angle (θ) qui est rendu plus grand que 15 degrés par rapport à l'axe (19) de l'élément cylindrique (2).

2. Seringue selon la revendication 1, dans laquelle la dérivation (20) a un sens longitudinal incliné par rapport à l'axe (19) de l'élément cylindrique (2).

3. Seringue selon la revendication 1, dans laquelle la dérivation (20) a une partie centrale dotée d'une partie courbe (23).

4. Seringue selon la revendication 1, comprenant une pluralité de dérivations dont une première dérivation (20a) sur le côté de la partie d'extrémité de base (4) et une seconde dérivation (20b) sur le côté de la partie d'extrémité frontale (3),
la première dérivation (20a) ayant une longueur dans un sens d'un axe (19) de l'élément cylindrique (2), laquelle est rendue plus courte que l'élément de bouchon intermédiaire (14), une longueur depuis une entrée (21a) de la première dérivation (20a) jusqu'à une sortie (22b) de la seconde dérivation (20b) étant formée plus longue que l'élément de bouchon intermédiaire (14),
l'élément de bouchon intermédiaire (14) ayant une surface périphérique externe concave pour fournir une rainure (24) qui fait communiquer la première dérivation (20a) avec la seconde dérivation (20b) quand l'élément de bouchon intermédiaire (14) a atteint une position où les dérivations (20) sont formées.

5. Seringue selon l'une quelconque des revendications 1 à 4, dans laquelle une surface d'extrémité de la surface interne de la dérivation (20) est située sur un côté d'une sortie (22) formée sur le côté de la partie d'extrémité frontale (3) et s'élève vers l'extérieur par un angle (θ') qui est plus grand que 45 degrés par rapport à l'axe (19) de l'élément cylindrique (2).
